# EUROPEAN PATENT APPLICATION

(11) **EP 4 368 693 A1**
(43) Date of publication of application: **15.05.2024**
(21) Application number: 22212702.9
(22) Date of filing: 12.12.2022
(51) Int. Cl.: C11D 3/38, C11D 3/48

(54) **METHOD TO PROVIDE LONG-LASTING CLEANING**

(30) Priority: 10.11.2022 EP 22206701
(71) Applicant: The Procter & Gamble Company, Cincinnati, OH 45202 (US)
(72) Inventor: BETTIOL, Jean-Luc Philippe, 1853 Strombeek-Bever (BE); CASERTA, Justin Angelo, Cincinnati, 45202 (US); CHARBONNEAU, Duane Larry, Cincinnati, 45202 (US); DESILVESTRO, Irene, 1853 Strombeek-Bever (BE); KONYA, Abigail Mary, Newcastle upon Tyne, NE12 9TS (GB); MAGNONI, Francesco, 1853 Strombeek-Bever (BE); TAVERNIER, Sarah, 1853 Strombeek-Bever (BE)
(74) Representative: P&G Patent Belgium UK

(57) **Abstract**

A method of treating an inanimate surface to provide long-lasting cleaning, including prevention of mold growth on the surface, the method comprises the step of treating the surface with a composition comprising at least 10²cfu/g of bacterial spores wherein the bacterial spores are selected from the group consisting of spores of *Bacillus amyloliquefaciens* strain D747 and a variant thereof, wherein the variant has at least 96% and preferably at least 99% whole genome sequence identity with *Bacillus amyloliquefaciens* strain D747.

## Description

### FIELD OF THE INVENTION

The present invention relates to a method to provide long-lasting cleaning to an inanimate surface using a composition comprising *Bacillus amyloliquefaciens* spores or a variant thereof. It also relates to a composition for use in the method of the invention and the use of the *Bacillus amyloliquefaciens* or a variant thereof to provide long-lasting cleaning to an inanimate surface.

### BACKGROUND OF THE INVENTION

It is desirable that the cleaning of inanimate surfaces not only happens momentarily but also that the cleaning continues over time. Preferably, the cleaning continues a few hours and even better a few days or weeks after the surface has been treated with a cleaning product. Long-lasting cleaning can contribute to the cleaning of soils that were not removed when the product was first applied, and it can also contribute to the removal of new soils that might appear over time. It is not only the removal of soils that is usually tackled in a cleaning process, it is also the removal of malodor. As in the case of soils, malodor might not be removed during the initial cleaning process or malodor can develop over time. Another problem faced by inanimate surfaces can be mold growth.

Mold tends to growth in humid and damp environments in most types of inanimate surfaces, including indoors and outdoor surfaces. Mold is particularly prone to growth in shower and bath areas, in particular on ceilings, tiles and grouts. The main type of mold found in kitchens and bathrooms seem to be black mold, for example *Cladosporium halotolerans, Exophiala* and *Aspergillus.* Mold is difficult to clean requiring time spent scrubbing and harsh cleaners. Mold can not only be difficult to remove but also difficult to prevent regrowth after a surface has been cleaned. There is a need to provide a method to provide long-lasting cleaning, including mold growth prevention.

### SUMMARY OF THE INVENTION

According to the first aspect of the invention there is provided a method of treating an inanimate surface to provide long-lasting cleaning, preferably on a kitchen or bathroom surface. The method comprises the step of treating the surface with a composition which comprises bacterial spores. The bacterial spores are selected from the group consisting of spores of *Bacillus amyloliquefaciens* strain D747 and a variant thereof, wherein the variant has at least 96%, preferably at least 97%, preferably at least 98%, preferably at least 99% and even more preferably at least 99.8% whole genome sequence identity with *Bacillus amyloliquefaciens* strain D747.

According to the second aspect of the invention, there is provided the use of bacterial spores selected from the group consisting of spores of *Bacillus amyloliquefaciens* strain D747 and a variant thereof, wherein the variant has at least 96%, preferably at least 97%, preferably at least 98%, preferably at least 99% and even more preferably at least 99.8% whole genome sequence identity with *Bacillus amyloliquefaciens* strain D747, to provide long-lasting cleaning, including prevention of mold, preferably black mold, growth on an inanimate surface.
According to the third aspect of the invention, there is provided a composition suitable for use in method of the invention.

The elements of the composition described in relation to the first aspect of the invention apply *mutatis mutandis* to the other aspects of the invention.

### DETAILED DESCRIPTION OF THE INVENTION

The present invention envisages a method of treating an inanimate surface, preferably a soft or a hard surface, to provide long-lasting cleaning, including cleaning and malodor removal/prevention over a sustained period of time and prevention of mold growth, preferably the present invention is directed to a method of treating an inanimate surface to provide prevention of black-mold growth. It also envisages a composition comprising specific bacterial spores, suitable for use in the method of the invention and the use of the specific bacterial spores to provide long-lasting cleaning.

As used herein, the articles including "the," "a" and "an" when used in a claim or in the specification, are understood to mean one or more of what is claimed or described.

As used herein, the terms "include," "includes" and "including" are meant to be non-limiting.

The term "inanimate surface" includes all types of non-alive surfaces such as soft and hard surfaces. The term "inanimate surface" excludes all types of alive surfaces such as plants, animals, etc. Soft surfaces include clothes, fabrics, etc. The term "hard surface" refers to a solid, substantially non-flexible surface such as a countertop, tile, floor, wall, panel, window, plumbing fixture, kitchen and bathroom furniture, appliance, engine, circuit board, and dish. Hard surfaces may include for example, health care surfaces, food processing surfaces, bathroom surfaces, and the like, and may be interior or exterior.

The term "long-lasting cleaning" means cleaning that happens during a sustained period of time after a surface is treated with a cleaning composition. By a "a sustained period of time" is understood, at least one hour, preferably at least one day and more preferably at least one week.

The term "substantially free of' or "substantially free from" as used herein refers to either the complete absence of an ingredient or a minimal amount thereof merely as impurity or unintended byproduct of another ingredient. A composition that is "substantially free" of/from a component means that the composition comprises less than about 0.01%, or less than about 0.001%, or even 0%, by weight of the composition, of the component.

In this description, all concentrations and ratios are on a weight basis of the composition unless otherwise specified.

Unless otherwise noted, all component or composition levels are in reference to the active portion of that component or composition, and are exclusive of impurities, for example, residual solvents or by-products, which may be present in commercially available sources of such components or compositions.

All measurements are performed at 20°C unless otherwise specified.

### Method of the invention

The present disclosure relates to a method of treating an inanimate surface to provide long-lasting cleaning. The surface is treated with a composition comprising bacterial spores.

### Composition

The composition of the present invention may be formulated as a concentrate or ready-to-use composition. The composition of the present invention may deliver cleaning and shine benefits on inanimate surfaces, both hard and soft surfaces. The composition of the present invention delivers improved long-lasting cleaning on hard and soft surfaces. The composition of the present invention may deliver cleaning benefits, shine benefits, malodor removal/prevention benefits as well as mold growth prevention on hard and soft surfaces. Preferably, the composition of the invention is a liquid composition, more preferably an aqueous liquid composition. As a concentrate execution, the composition comprises less than 80% by weight of the composition of water, preferably from 40% to 60% by weight of the composition of water. As a ready-to-use execution, the composition comprises more than 80% by weight of the composition of water, preferably from 90% to 99% by weight of the composition of water.

Concentrates may be diluted with water in order to provide an in-use solution having a desired level of cleaning properties.

The water used to dilute the concentrate (water of dilution) can be available at the locale or site of dilution. The water of dilution may contain varying levels of hardness depending upon the locale. Service waters available from various municipalities have varying levels of hardness. It is desirable to provide a concentrate that can handle the hardness levels found in the service water of various municipalities. The water of dilution may have a hardness ranging from about zero to at least about 400 ppm hardness (as CaCO₃).

Concentrated solutions may provide for improved economics to the manufacturer and to the user as they may comprise less water and may use less packaging material on a per-use basis, as compared to ready-to-use compositions. A concentrated composition may be diluted with water at a weight ratio of composition to water ranging from about 1:1.5 to about 1:250, or from about 1:4 to about 1:100. The terms "in-use composition" or "in-use diluted composition" refer to concentrated compositions that have been diluted with water prior to use.
Alternatively, the compositions may be in a ready-to-use form, preferably to be delivery in spray form.

The pH of the composition helps with spore stability. The pH of the composition is preferably less than 11.5, preferably from 4 to 9 as measured at 20°C. Accordingly, the compositions herein can comprise a pH adjusting agent.

### Bacterial spores

The biological component used in the present invention are bacterial spores as opposed to vegetative cells. The bacterial spores are bacterial spores of *Bacillus amyloliquefaciens* or a variant thereof, in particular the bacterial spores are selected from the group consisting of:
i) spores of *Bacillus amyloliquefaciens* strain D747; and
ii) spores of a variant of *Bacillus amyloliquefaciens* strain D747, wherein the variant has at least 96%, preferably 97%, more preferably 98%, more preferably 99% and especially at least 99.8% whole genome sequence identity with *Bacillus amyloliquefaciens* strain D747.

In order to determine the genome identity between a variant and *Bacillus amyloliquefaciens* strain D747, the whole genome of the variant can be sequenced using for example, either PacBio Sequel, Oxford Nanopore Minion, or Illumia NextSeq 500 with pair-end reads 2x150 bp sequencing platforms. PacBio Sequel is preferred for use herein. Assembly is performed using a combination of 5 assemblers: Canu, SPAdes, Flye, Raven, and/or Unicycler. The genome is assembled with the hybrid mode of Illumina short-reads and Nanopore long-reads, and then used for the genomic average nucleotide identity (ANI) calculation using OrthoANI software. The genomic ANI comparison is used to compare the variant to *Bacillus amyloliquefaciens* D747 as a reference. Sequence identity of >96% indicated the same species; while sequence identity of >99.98% indicates the same strain.

Preferably, the bacterial spores for use herein should produce, once they germinate, at least one cyclic lipopeptide from the chemical families of iturin, fengycin, and/or surfactin. Additionally, the preferred bacterial spores should comprise genes of the polyketide synthesis pathway (*pks*) in order to produce the cyclic lipopeptides iturin, fengycin, and/or surfactin.

It has to be noted that some strains which were originally considered to belong to *Bacillus subtilis* have now been identified to belong to *Bacillus amyloliquefaciens* (such as D747 and QST713).

*Bacillus amyloliquefaciens* (previously *Bacillus subtilis*) D747 strain has been described as a biological control agent useful for controlling a number of plant diseases, including, but not limited to, fungal diseases. U.S. Patent No. 7,094,592 to Watanabe et al., incorporated herein by reference in its entirety, describes the biological characteristics of the *Bacillus amyloliquefaciens* D747 strain, including bacteriological characteristics in accordance with Bergey's Manual of Systematic Bacteriology, Volume 1 (1984). U.S. Patent Publication No. 2013/0236522 to Misumi describes use of *amyloliquefaciens* D747 strain in formulations such as a dust, granule or powder. The *amyloliquefaciens* D747 strain was deposited at the National Institute of Advanced- Industrial Science and Technology, International Patent Organism Depositary as "Bacillus sp. D747" with Accession Number 'TERM P-18128", and was then transferred to be deposited under the Budapest Treaty on Nov. 8, 2002, as "Bacillus sp. D747" with new Accession Number "FERM BP-8234". QST713 is available as the commercial product SERENADE^{®} from AgraQuest, Inc., USA. Other strains suitable for use herein include *Bacillus subtilis* GB03, *Bacillus velezensis* FZB42 (from FZB Biotechnik GmbH, Berlin, Germany, Cot1, CL27), *Bacillus velezensis* BGSC 10A19, *Bacillus velezensis* BGSC 10A20 and *Bacillus velezensis* BGSC 10A21.

The composition of the invention comprises at least 10²cfu/g of bacterial spores, preferably at least 10⁴cfu/g of bacterial spores, more preferably from about 10⁴cfu/g to 10⁸cfu/g of bacterial spores.

The germinated cells from the spores are used to clean and to facilitate stain removal from a surface during a subsequent cleaning process. After a surface has been treated with the bacterial spores of the invention and the spores germinate, stains deposited on that surface are more easily removed than without previous treatment. This effect is generally referred to as "next time cleaning benefit". The effect can be especially noticeable on enzymatic stains, stains comprising a carbohydrate and/or a protein and/or a fat. The spores can also contribute to malodour removal and to mold growth prevention, especially black mold growth prevention.

### Surfactant

The composition of the invention preferably comprises surfactants, more preferably from 0.01% to 10%, preferably from 0.05% to 8%, more preferably from 0.08% to 5%, more preferably 0.1% to 3% by weight of the composition of surfactant. The surfactant also contributes to the cleaning and/or shine provided by the composition.

### Alkoxylated alcohol nonionic surfactants

Suitable alkoxylated alcohol nonionic surfactants are according to the formula RO-(A)ₙH, wherein: R is a primary C₄ to C₁₈, preferably a C₆ to C₁₆, more preferably a C₁₀ to C₁₆ and even more preferably from C₁₂ to C₁₄ branched or linear alkyl chain, or a C₆ to C₂₈ alkyl benzene chain; A is an ethoxy or propoxy or butoxy unit, or mixtures thereof, and wherein n is an integer from 1 to 30, preferably from 1 to 15, more preferably from 3 to 12 even more preferably from 3 to 8. Preferred R chains for use herein are the C₁₀ to C₁₆ linear or branched alkyl chains. Especially preferred for use herein is an alcohol alkoxylated having a C₁₂-C₁₄ chain length and having from 8 to 10 ethoxy groups.

Suitable branched alkoxylated alcohols may be selected from the group consisting of: C₄-C₁₀ alkyl branched alkoxylated alcohols, and mixtures thereof. The branched alkoxylated alcohol can be derived from the alkoxylation of C₄-C₁₀ alkyl branched alcohols selected form the group consisting of: C₄-C₁₀ primary mono-alcohols having one or more C₁-C₄ branching groups.

By C₄-C₁₀ primary mono-alcohol, it is meant that the main chain of the primary mono-alcohol has a total of from 4 to 10 carbon atoms. The C₄-C₁₀ primary mono-alcohol can be selected from the group consisting of: methyl butanol, ethyl butanol, methyl pentanol, ethyl pentanol, methyl hexanol, ethyl hexanol, propyl hexanol, dimethyl hexanol, trimethyl hexanol, methyl heptanol, ethyl heptanol, propyl heptanol, dimethyl heptanol, trimethyl heptanol, methyl octanol, ethyl octanol, propyl octanol, butyl octanol, dimethyl octanol, trimethyl octanol, methyl nonanol, ethyl nonanol, propyl nonanol, butyl nonanol, dimethyl nonanol, trimethyl nonanol and mixtures thereof.

The C₄-C₁₀ primary mono-alcohol can be selected from the group consisting of: ethyl hexanol, propyl hexanol, ethyl heptanol, propyl heptanol, ethyl octanol, propyl octanol, butyl octanol, ethyl nonanol, propyl nonanol, butyl nonanol, and mixtures thereof. Preferably the C₄-C₁₀ primary mono-alcohol is selected from the group consisting of: ethyl hexanol, propyl hexanol, ethyl heptanol, propyl heptanol, and mixtures thereof.

The C₄-C₁₀ primary mono-alcohol is most preferably ethyl hexanol, and propyl heptanol. Especially preferred for use herein are ethoxylated ethyl hexanol comprising from 4 to 10 ethoxy groups.

In the branched alkoxylated alcohol, the one or more C₁-C₄ branching group can be substituted into the C₄-C₁₀ primary mono-alcohol at a C1 to C3 position, preferably at the C1 to C2 position, more preferably at the C2 position, as measured from the hydroxyl group of the starting alcohol.

The branched alkoxylated alcohol can comprise from 1 to 14, preferably from 2 to 7, more preferably from 4 to 6 ethoxylate units, and optionally from 1 to 9, preferably from 2 to 7, more preferably from 4 to 6 of propoxylate units.

The branched alkoxylated alcohol is preferably 2-ethyl hexan-1-ol ethoxylated to a degree of from 4 to 6, and propoxylated to a degree of from 4 to 6, more preferably, the alcohol is first propoxylated and then ethoxylated. Another preferred branched alkoxylated alcohols are 2-alkyl-1-alkanols such as alkoxylated C₁₀ guerbet alcohols with 1 to 14, preferably from 2 to 7, more preferably from 3 to 6 ethoxylate or ethoxylate-propoxylate units.

Non-limiting examples of suitable branched alkoxylated alcohols are, for instance, Ecosurf^{®} EH3, EH6, and EH9, commercially available from DOW, and Lutensol^{®} XP alkoxylated Guerbet alcohols & Lutensol^{®} XL ethoxylated Guerbet alcohols available from BASF.

Linear alcohol alkoxylated nonionic surfactants preferred herein are alkoxylated nonionic surfactants with a C₈, C₁₀, C₁₂, mixtures of C₈ to C₁₀, mixtures of C₁₀ to C₁₂, mixtures of C₉ to C₁₁ linear alkyl chain and 8 or less ethoxylate units, preferably 3 to 8 ethoxylate units.

Non-limiting examples of suitable linear alkoxylated nonionic surfactants for use herein are Dobanol^{®} 91-2.5 (R is a mixture of C₉ and C₁₁ alkyl chains, n is 2.5), Dobanol^{®} 91-5 (R is a mixture of C₉ to C₁₁ alkyl chains, n is 5); Dobanol^{®} 91-10 (R is a mixture of C₉ to C₁₁ alkyl chains, n is 10); Greenbentine DE60 (R is a C₁₀ linear alkyl chain, n is 6); Marlipal 10-8 (R is a C₁₀ linear alkyl chain, n is 8); Neodol 91-8 (R is a mixture of C₉ to C₁₁ alkyl chains, n is 8); Empilan^{®} KBE21 (R is a mixture of C₁₂ and C₁₄ alkyl chains, n is 21); Lutensol ON30 (R is C₁₀ linear alkyl chain, n is 3); Lutensol ON50 (R is C₁₀ linear alkyl chain, n is 5); Lutensol ON70 (R is C₁₀ linear alkyl chain, n is 7); Novel 610-3.5 (R is mixture of C₆ to C₁₀ linear alkyl chains, n is 3.5); Novel 810FD-5 (R is mixture of C₈ to C₁₀ linear alkyl chains, n is 5); Novel 10-4 (R is C₁₀ linear alkyl chain, n is 4); Novel 1412-3 (R is mixture of C₁₂ to C₁₄ linear alkyl chains, n is 3); Lialethl^{®} 11-5 (R is a C₁₁ linear alkyl chain, n is 5); Lialethl^{®} 11-21 (R is a mixture of linear and branched C₁₁ alkyl chain, n is 21), or mixtures thereof.

The alkoxylated nonionic surfactant may be a secondary alcohol ethoxylate such as for example the Tergitol^{™}-15-S surfactants having the general formula shown below and commercially available from DOW 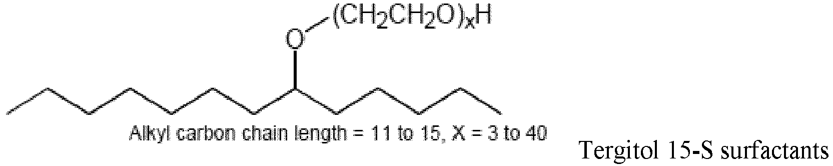 Preferred secondary alcohol ethoxylate surfactants have 3-9 EO units.

Another suitable alkoxylated nonionic surfactant is an alkyl ethoxy alkoxy alcohol, preferably wherein the alkoxy part of the molecule is propoxy, or butoxy, or propoxy-butoxy. More preferred alkyl ethoxy alkoxy alcohols are of formula (II): 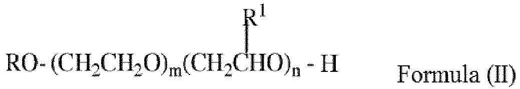 wherein:
R is a branched or unbranched alkyl group having 8 to 16 carbon atoms;
R¹ is a branched or unbranched alkyl group having 1 to 5 carbon atoms;
n is an integer from 1 to 10; and m is an integer from 6 to 35.

R is preferably from 12 to 15, preferably 13 carbon atoms. R¹ is preferably a branched alkyl group having from 1 to 2 carbon atoms. n is preferably an integer from 1 to 5. m is preferably an integer from 8 to 25. Preferably, the weight average molecular weight of the ethoxylated alkoxylated nonionic surfactant of formula (II) is from 500 to 2000g/mol, more preferably from 600 to 1700 g/mol, most preferably 800 to 1500 g/mol.

The ethoxylated alkoxylated nonionic surfactant can be a polyoxyalkylene copolymer. The polyoxyalkylene copolymer can be a block-heteric ethoxylated alkoxylated nonionic surfactant, though block-block surfactants are preferred. Suitable polyoxyalkylene block copolymers include ethylene oxide/propylene oxide block polymers, of formula (III):

(EO)ₓ(PO)_{y}(EO)ₓ,

or

(PO)ₓ(EO)_{y}(PO)ₓ

wherein EO represents an ethylene oxide unit, PO represents a propylene oxide unit, and x and y are numbers detailing the average number of moles ethylene oxide and propylene oxide in each mole of product. Such materials tend to have higher molecular weights than most non-ionic surfactants, and as such can range between 1000 and 30000 g/mol, although the molecular weight should be above 2200 and preferably below 13000 to be in accordance with the invention. A preferred range for the molecular weight of the polymeric non-ionic surfactant is from 2400 to 11500g/mol. BASF (Mount Olive, N.J.) manufactures a suitable set of derivatives and markets them under the Pluronic trademarks. Examples of these are Pluronic (trademark) F77, L62 and F88 which have the molecular weight of 6600, 2450 and 11400 g/mol respectively.

Other suitable ethoxylated alkoxylated nonionic surfactants are described in Chapter 7 of Surfactant Science and Technology, Third Edition, Wiley Press, ISBN 978-0-471-68024-6.

Most preferably the alkoxylated nonionic surfactant is selected from the group consisting of: 2-propylheptyl EO8 (Lutensol XL89-BASF); 2-propylheptyl EO5 (Lutensol XL50-BASF); C₁₀ alcohol EO5 (Lutensol ON 50-BASF); C₁₀ -alcohol EO7 (Lutensol ON 70-BASF); C₈-C₁₀ EO5 (Novel 810 FD5 Sasol); C₁₀ EO4 (Novel 10-4 Sasol); Tergitol 15-S-3; Tergitol 15-S-5; Tergitol 15-S-7; and Ethyl hexanol PO5EO6 (Ecosurf EH6-Dow).

### Alkyl polyglycosides

Alkyl polyglycosides are biodegradable nonionic surfactants which are well known in the art and can be used in the compositions of the present invention. Suitable alkyl polyglycosides can have the general formula CₙH₂ₙ₊₁O(C₆H₁₀O₅)ₓH wherein n is preferably from 8 to 16, more preferably 8 to 14, and x is at least 1. Examples of suitable alkyl polyglucoside surfactants are the TRITON^{™} alkyl polyglucosides from Dow; Agnique PG, Disponil APG, Glucopon alkyl polyglucosides from BASF and Plantaren 2000 from BASF. Preferred alkyl polyglucoside surfactants are those where n is 8 to 12, more preferably 8 to 10, such as for example Triton^{™} CG50 (Dow).

### Alkyl amine oxide

Suitable amine oxide surfactants include: R₁R₂R₃NO wherein each of R₁, R₂ and R₃ is independently a saturated or unsaturated, substituted or unsubstituted, linear or branched hydrocarbon chain having from 1 to 30 carbon atoms. Preferred amine oxide surfactants are amine oxides having the following formula: R₁R₂R₃NO wherein R1 is a hydrocarbon chain comprising from 1 to 30 carbon atoms, preferably from 6 to 20, more preferably from 8 to 16 and wherein R₂ and R₃ are independently saturated or unsaturated, substituted or unsubstituted, linear or branched hydrocarbon chains comprising from 1 to 4 carbon atoms, preferably from 1 to 3 carbon atoms, and more preferably are methyl groups. R1 may be a saturated or unsaturated, substituted or unsubstituted linear or branched hydrocarbon chain.

Highly preferred amine oxides are C₈ dimethyl amine oxide, C₁₀ dimethyl amine oxide, C₁₂ dimethyl amine oxide, , C₁₄ dimethyl amine oxide, and mixtures thereof C₈ dimethyl amine oxide is commercially available under the trade name Genaminox^{®} OC from Clariant; C₁₀ dimethyl amine oxide is commercially available under the trade name Genaminox^{®} K-10 from Clariant; C₁₂ dimethyl amine oxide is commercially available under the trade name Genaminox^{®} LA from Clariant and of Empigen OB from Huntsman; C₁₄ amine oxide is commercially available under the trade name of Empigen OH 25 from Huntsman. Other suitable amine oxide surfactants are cocoyldiethoxy amine oxide available under the trade name of Genaminox CHE from Clariant, and cocamydopropyl amine oxide commercially available under the trade name of Empigen OS/A from Huntsman. Particularly preferred amine oxide surfactants are C₁₀ dimethyl amine oxide such as Genaminox K-10.

### Alkyl glucamide surfactants

The composition of the invention may comprise an alkyl glucamide surfactant. Glucamide surfactants are non ionic surfactants in which the hydrophilic moiety (an amino-sugar derivative) and the hydrophobic moiety (a fatty acid) are linked via amide bonds. This results in a chemical linkage, which is highly stable under alkaline conditions. Particularly preferred alkyl glucamide surfactants are N-alkyl-N-acylglucamides of the formula (III):

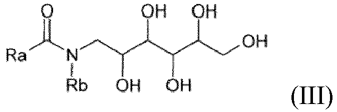

Wherein Ra is a linear or branched, saturated or unsaturated hydrocarbyl group having 6 to 22 carbon atoms, and Rb is a C₁-C₄ alkyl group. Particularly preferably, Rb in formula (III) is a methyl group. Non-limiting examples of these glucamide surfactants are: N-octanoyl-N-methylglucamide, N-nonanoyl-N-methylglucamide, N-decanoyl-N-methylglucamide, N-dodecanoyl-N-methylglucamide, N-cocoyl-N-methylglucamide, available under the trade name of GlucoPure Foam from Clariant, N-lauroyl/myristoyl-N-methylglucamide, (available under the trade name of GlucoPure Deg from Clariant, and N-octanoyl/decanoyl-N-methylglucamide, available under the trade name of GlucoPure Wet by Clariant.

Alkyl glucamine surfactants are suitable for the composition of the invention.

These surfactants are described in EP16184415 and US20190055496.

### Zwitterionic and amphoteric surfactants

The hard surface cleaning composition may comprise an amphoteric surfactant, a zwitterionic surfactant, and mixtures thereof. Suitable zwitterionic surfactants typically contain both cationic and anionic groups in substantially equivalent proportions so as to be electrically neutral at the pH of use, and are well known in the art. Some common examples of zwitterionic surfactants are described in US. Pat. Nos. 2,082,275, 2,702,279 and 2,255,082.

Suitable zwitteronic surfactants include betaines such alkyl betaines, alkylamidobetaine, amidazoliniumbetaine, sulfobetaine (INCI Sultaines) as well as the phosphobetaine.

Suitable betaines are the alkyl betaines of the formula (Ia), the alkyl amido betaine of the formula (Ib), the sulfo betaines of the formula (Ic) and the amido sulfobetaine of the formula (Id);

R1-N+(CH3)2-CH2COO- (Ia)

RI-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R1 is a saturated or unsaturated C₆-C₂₂ alkyl residue, preferably C₈-C₁₈ alkyl residue. Particularly preferred are betaines of the formula Ia such as for example N-alkyl-N-dimethyl betaine like the one sold under the trade name of Empigen^{®} BB by Huntsman.

If the composition comprises a zwitterionic surfactant, it is preferably a betaine of the formula Ia such as for example N-alkyl-N-dimethyl betaine like the one sold under the trade name of Empigen BB by Huntsman. It has been found these betaines greatly increase the antibacterial efficacy of the modified amino acids.

Amphoteric surfactants can be either cationic or anionic depending upon the pH of the composition. Suitable amphoteric surfactants include the products sold under the trade name Miranol by Solvay-Novecare such as, for example, sodium lauroamphoacetate (Miranol Ultra L-32E), sodium stearoampho acetate (Miranol DM), disodium cocoamphodiacetate (Miranol C2m Conc NP), disodium lauroamphodiacetate (Miranol BM Conc), disodium capryloampho dipropionate (Miranol JBS), sodium mixed C₈ amphocarboxylate (Miranol JEM Conc), and sodium capryloampho hydroxypropyl sulfonate (Miranol JS). Other non-limiting examples of suitable amphoteric surfactants are disodium capryloamphodiacetate (Mackam 2CY 75-Solvay Novecare), octyliminodipropionate (Ampholak YJH40-Akzo Nobel), sodium lauriminodipropionate (Mirataine H2C-HA-Solvay Novecare), and sodium lauroamphohydroxypropylsulfonate (Mackam LS- Solvay Novecare

Other suitable additional surfactants can be found in McCutcheon's Detergents and Emulsifiers, North American Ed. 1980.

### Cationic Surfactant

The compositions disclosed herein may comprise a cationic surfactant. Non-limiting examples of cationic surfactants include: the quaternary ammonium surfactants, which can have up to 26 carbon atoms and may include alkoxylate quaternary ammonium (AQA) surfactants, dimethyl hydroxyethyl quaternary ammonium, and/or dimethyl hydroxyethyl lauryl ammonium chloride; polyamine cationic surfactants; cationic ester surfactants; amino surfactants, e.g., amido propyldimethyl amine (APA); and mixtures thereof.

Suitable cationic surfactants also may include alkyl pyridinium compounds, alkyl quaternary ammonium compounds, alkyl quaternary phosphonium compounds, alkyl ternary sulphonium compounds, and mixtures thereof.

Suitable cationic detersive surfactants are quaternary ammonium compounds having the general formula:

(R)(R₁)(R₂)(R₃)N⁺ X⁻

wherein, R is a linear or branched, substituted or unsubstituted C₆₋₁₈ alkyl or alkenyl moiety, R₁ and R₂ are independently selected from methyl or ethyl moieties, R₃ is independently selected from a linear or branched, substituted or unsubstituted C₆₋₁₈ alkyl or alkenyl moiety or a hydroxyl, hydroxymethyl or a hydroxyethyl moiety, X is an anion which provides charge neutrality, suitable anions include: halides, for example chloride; sulphate; and sulphonate. Suitable cationic surfactants are mono-C₆₋₁₈ alkyl mono-hydroxyethyl di-methyl quaternary ammonium chlorides. Highly suitable cationic surfactants are di-C₈₋₁₀ alkyl di-methyl quaternary ammonium chloride, mono-C₁₆ alkyl tri-methyl quaternary ammonium chloride, di-C₁₀₋₁₂ alkyl di-methyl quaternary ammonium chloride and mono-Cio alkyl mono-hydroxyethyl di-methyl quaternary ammonium chloride.

Particularly preferred surfactants for use herein include nonionic surfactants, in particular branched alcohol alkoxylates, more in particular 2-ethyl hexan-1-ol ethoxylated to a degree of from 4 to 6, and propoxylated to a degree of from 4 to 6, more preferably, the alcohol is first propoxylated and then ethoxylated, and 2-alkyl-1-alkanols such as alkoxylated C₁₀ guerbet alcohols with 1 to 14, preferably 2 to 8, more preferably 3 to 6 ethoxylate or ethoxylate-propoxylate units. Other particularly preferred non-ionic surfactants include linear alcohol alkoxylated nonionic surfactants with C₈, C₁₀, C₁₂, C₁₄ mixtures of C₈ to C₁₀, mixtures of C₁₀ to C₁₂, mixtures of C₁₂ to C₁₄, mixtures of C₉ to C₁₁ linear alkyl chain and 10 or less ethoxylate units, preferably 3 to 9 ethoxylate units. Most preferably the alkoxylated nonionic surfactant is selected from the group consisting of: C₁₂-C₁₄ alcohol EO9 (Surfonic L 24-9), 2-propylheptyl EO8 (Lutensol XL89-BASF); 2-propylheptyl EO5 (Lutensol XL50-BASF); C₁₀ alcohol EO5 (Lutensol ON 50-BASF); C₁₀ alcohol EO7 (Lutensol ON 70-BASF); C₈-C₁₀ alcohol EO5 (Novel 810 FD5 Sasol); C₁₀ alcohol EO4 (Novel 10-4 Sasol); and 2-ethyl-hexanol PO5EO6 (Ecosurf EH6-Dow).

Other particularly preferred surfactants for use here in include linear amine oxide surfactants, in particular C₈-C₁₂ dimethyl amine oxide, more in particular C₁₀ dimethyl amine oxide; alkyldimethylbetaine surfactants, more in particular N,N-Dimethyl-N-dodecylglycine betaine (Empigen BB-Huntsman); alkyl glucamide surfactants such as N-alkyl-N-acylglucamide preferably N-decanoyl-N-methylglucamine, and the alkyl glucamide surfactants sold under the name of GlucoPure^{®}, GlucoTain^{®}, and GlucoWet^{®} by Clariant; alkylpolyglucoside surfactants, more in particular C₈ to C₁₂ alkyl polyglucosides, more preferably C₈ to C₁₀ alkyl polyglucosides such as for example Triton CG50 (Dow)

### Perfume

The composition comprises a perfume. The perfume is a mixture of odorant perfume raw materials, such as aromatic natural oils and aromatic chemicals, which taken together form a complex scent that delivers a number of benefits. These benefits may include the coverage of product base odor, scenting the product itself, and lingering scent radiating from the surface into the air after cleaning. When the composition is sprayed, the benefit may also include the delivery of scent to the air when spraying the composition on a surface, and the delivery of scent to the air while wiping the composition on the surface. The perfume may comprise at least 3, at least 5, at least 7, at least 11, or at least 15 perfume raw materials.

The perfume raw materials of the perfume may comprise at most 50%, or at most 40%, or at most 30%, for example from 0% to 20%, or from 0.01% to 10%, or from 0.02% to 5%, per weight of perfume raw materials comprising an α, β-unsaturated aldehyde function, an α, β-unsaturated ketone function, and/or an ester function.

For the purpose of the invention, an aromatic aldehyde/ketone wherein the aromatic ring is adjacent to the aldehyde or ketone group (e.g. anisic aldehyde or methyl β-naphthyl ketone) is considered as an α, β-unsaturated aldehyde/ketone.

The perfume raw materials of the perfume of the composition of the invention may comprise at most 50%, or at most 40%, or at most 30% for example from 0% to 20%, or from 0.01% to 10%, or from 0.02% to 5% per weight of perfume raw materials selected from benzyl acetate, methyl salicylate, allyl amyl glycolate, benzyl propionate, pomarose, methyl dihydrojasmonate, heliotropin, anisic aldehyde, delta damascone, amyl butyrate, iso-amyl isobutyrate, b-ionone, carvone, iso-butyl iso butanoate, methyl b-naphtyl ketone, citronellyl butyrate, iso-propyl miristate.

The perfume raw materials of the perfume of the composition of the invention may comprise at least 20% per weight, in particular at least 30%, or at least 40%, or at least 50%, or at least 60%, or at least 70% for example from 80% to 100%, or from 90% to 99.9% per weight of perfume raw materials comprising an α, β-saturated aldehyde function, an α, β-saturated ketone function, an alcohol function, an ether function, a nitrile function, and/or being a terpene or terpenoid.
For the purpose of the invention an α, β-saturated aldehyde function is an aldehyde function without unsaturation in the α and/or β position.

For the purpose of the invention an α, β-saturated ketone function is a ketone function without unsaturation in the α and/or β position.

The perfume raw materials of the perfume of the composition of the invention may comprise at least 20% per weight, in particular at least 30%, or at least 40%, or at least 50%, or at least 60%, or at least 70% for example from 80% to 100%, or from 90% to 99.9% per weight of perfume raw materials which do not comprise α, β-unsaturated aldehyde function, an α, β-unsaturated ketone function, and/or an ester function.

The perfume raw materials of the perfume of the composition of the invention may comprise at least 20% per weight, in particular at least 30%, or at least 40%, or at least 50%, or at least 60%, or at least 70% for example from 80% to 100%, or from 90% to 99.9% per weight of perfume raw materials which comprise α, β-saturated aldehyde function, an α, β-saturated ketone function, an alcohol function, an ether function, a nitrile function, and/or are a terpene and which do not comprise an α, β-unsaturated aldehyde function, an α, β-unsaturated ketone function, and/or an ester function.

The perfume raw materials of the perfume of the composition of the invention may comprise at least 20% per weight, in particular at least 30%, or at least 40%, or at least 50%, or at least 60%, or at least 70% for example from 80% to 100%, or from 90% to 99.9% per weight of perfume raw materials selected from d-muscenone 1, ambrox, polysantol, phenylethyl dimethyl carbinol, hydroxycitronellal, undecavertol, citronellol, linalool, p-cresyl methyl ether, cis-3-hexenol, clonal, limonene, tobacarol 2, tobacarol 3, tobacarol 1, b-naphthyl methyl ether. Other perfumes suitable for use in the composition of the invention are described in EP 1 493 803 A1 and WO 2002/06437 A1.

The composition may comprise from 0.01% to 5%, or from 0.2% to 4%, or even from 0.3 % to 4% of perfume by weight of the composition.

### pH adjusting agents

Depending on the targeted uses, a composition of the present invention for home care use may need appropriate pH conditions. For example, if the composition is used in the kitchen area, a high pH product may be desired in order to effectively remove grease soils commonly found in the area. If the composition is used in a bathroom area, soap scum and hard water deposits may be the primary concern. In such cases, a low pH product may be more appropriate. There is no limitation on the types of pH adjusting agents that can be added into the liquid composition of the present invention. Example of pH adjusting agents that can be used include, but are not limited to, triethanolamine, diethanolamine, monoethanolamine, sodium hydroxide, sodium carbonate, ammonium hydroxide, potassium hydroxide, potassium carbonate, calcium carbonate, citric acid, acetic acid, hydrochloric acid, sulfamic acid, sulfuric acid and the like. Preferably, alkaline compositions comprise from 0.1 to 2% of alkanol amine, more preferably monoethanolamine. Preferably, acid compositions comprise from 0.1 to 5% of an organic acid, preferably citric acid. Ammonium hydroxide is also preferred. It provides good shine.

### Other optional ingredients

The composition of the invention may comprise a variety of other optional ingredients depending on the technical benefit aimed for and the surface treated. Suitable optional ingredients for use herein include builders, other polymers, buffers, hydrotropes, colorants, stabilisers, radical scavengers, abrasives, soil suspenders, brighteners, anti-dusting agents, dispersants, dye transfer inhibitors, pigments, silicones and/or dyes.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

### Example 1: Mold growth inhibition on agar

*Bacillus amyloliquefaciens* (*B. amyloliquefaciens*) QST 713 (isolated from Serenade^{®} ASO) and *B. amyloliquefaciens* D747 (isolated from Revitalize^{®}) bacterial cultures were prepared as detailed herein below. Liquid mold cultures *of Cladosporium halotolerans (C. halotolerans), Rhinocladiella similis* (*R. similis*) and *Pleosporales* (all isolates from bathrooms and gene sequences identified through metagenomics analysis) were prepared as detailed herein below. Mold inhibition on agar was visually assessed for both bacterial strains against all three mold isolates. Both *B. amyloliquefaciens* QST 713 and *B. amyloliquefaciens* D747 provide mold growth inhibition on agar against all 3 isolates tested (Table 1). Other strains were tested vs *C*. *halotolerans* as illustrated as well in Table 1.

| **Table 1: Mold growth inhibition efficacy of Biotic strains on agar** | | | |
|---|---|---|---|
| **Biotic Strain** | **Mold Strain** | **Mold Inhibition Seen** | **Identity to D747** |
| *B. amyloliquefaciens* QST 713 | *C. halotolerans* | Yes | 97.62% |
| | *R. similis* | Yes | |
| | *Pleosporales* | Yes | |
| *B. amyloliquefaciens* D747 | *C. halotolerans* | Yes | 100% |
| | *R. similis* | Yes | |
| | *Pleosporales* | Yes | |
| *GB03* | *C. halotolerans* | Yes | 97.73% |
| FZB42 | *C. halotolerans* | Yes | 97.67% |
| CH61 | *C. halotolerans* | Yes | 97.77% |
| HB6 | *C. halotolerans* | Yes | 97.81% |
| *Bacillus atrophaeus* ATCC 9372 | *C. halotolerans* | No | 77.44% |
| *Bacillus badius* ATCC 14574 | *C. halotolerans* | No | 67.42% |
| *Bacillus coagulans* ATCC 7050 | *C. halotolerans* | No | 67.90% |
| *Bacillus licheniformis* ATCC 12759 | *C. halotolerans* | No | 72.46% |
| *Bacillus licheniformis* ATCC 14580 | *C. halotolerans* | No | 72.61% |
| *Bacillus megaterium* ATCC 14581 | *C. halotolerans* | No | 67.57% |
| *Bacillus subtilis* ATCC 11774 | *C. halotolerans* | No | 77.19% |
| *Bacillus subtilis* ATCC 6051 | *C. halotolerans* | No | 76.87% |

### Test method:

A liquid bacterial culture is created by adding a loop full of culture grown on a solid media into the desired liquid media, mixing it and leaving it to grow at ideal time and temperature. SDA (Sabouraud Dextrose Agar) is heated to melt and allowed to cool to ~50°C. SDA is poured into a large tube for dispensing (~20ml SDA required per petri dish). A liquid mold suspension is prepared by taking a culture plate with mold grown for 5-7 days, followed washing it with 10mL saline Tween and wiping the surface with a swab to disrupt spores. Once spores are suspended, the mixture is filtered through a cell strainer into a 50mL conical tube. The liquid mold suspension is diluted 1:250 and added to the cooled SDA. The SDA containing mold suspension is poured into clean sterile petri dishes and left to harden. Using the wide end of a 1mL pipette tip, a well is made into the centre of the plate a plug of agar removed. 50µL TSB (Tryptic Soy Broth), or chosen growth media, is added to the well followed by 5µL of bacterial culture. Plates are incubated at 28°C with 95% RH (relative humidity). Plates are observed for halo of inhibition in mold growth.

### Example 2: Mold growth inhibition on gypsum

*B. amyloliquefaciens* QST 713 and *B. amyloliquefaciens* D747 bacterial suspensions containing *10⁷ cfu*/*mL* were added to the surface of gypsum pellets, as detailed herein below, and incubated for 24-28h. A liquid mold culture *of C. halotolerans* was created following the method specified herein below. Mold inhibition on gypsum was assessed for both bacterial strains against *C*. *halotolerans* through visual assessment. *B. amyloliquefaciens* QST 713 does not provided visual mold inhibition against *C*. *halotolerans* on gypsum. *B. amyloliquefaciens* D747 provides visual mold growth inhibition against *C*. *halotolerans* on gypsum (Table 2).

| **Table 2: Mold growth inhibition on gypsum** | | |
|---|---|---|
| **Biotic Strain** | **Mold Strain** | **Mold Inhibition Seen** |
| *B. subtilis* QST 713 | *C. halotolerans* | No |
| *B. amyloliquefaciens* D747 | *C. halotolerans* | Yes |

### Test method:

A silicone mould recipient is autoclaved and cleaned with 70% ethanol. 20mL of sterile deionised (DI) water and 20mL sterile PDB (Potato Dextrose Broth) are added to 80g of gypsum and mixed thoroughly using a sterile rod until a homogenous paste is created. A sterile syringe is used to aspirate and distribute the gypsum paste into the silicone mould. Pellets are formed and allowed to dry at room temperature for 24-48h. Once dry, gypsum pellets are transferred into the wells of a sterile 12-well plate. 1mL sterile DI water is used to wet the surface of each gypsum pellet, this created a humid environment in the MTP (microtiter plate) during incubation. 100µL of a bacterial spore suspension with the desired concentration or control solutions (e.g water) is added to the gypsum pellet. MTP containing gypsum pellets is inoculated with bacteria or control incubated for the desired incubation time (e.g. 48h) at 28°C and 95% RH. Taking a pure culture of the chosen mold (e.g. *Cladosporium halotolerans*) grown on PDA (potato dextrose agar) for 7-14 days, 3mL of Tween 80 solution are added to the plate. Using an inoculation loop, growth is carefully scraped to release the spores into the suspension. The spore suspension is collected in a sterile conical tube and vortexed until the suspension is homogeneous. The spore suspension is filtered using a 40µm cell strainer, into a sterile 50mL conical tube. The suspension is diluted 1 in 50 in sterile DI water created and the number of spores present is calculated using a cell counting chamber and a microscope (40x objective). The number of spores in the suspension is calculated by first counting three grids inside of cell counting chamber, second making an average of the counts then finally calculating final level by multiplying average by 10⁴, to take into account the cell counting chamber, and by 50 for dilution factor. The suspension is diluted to give a final mold suspension containing 10⁵ cells/mL. 100µL of the mold suspension is added to the surface of the gypsum pellets (already inoculated with bacteria or control) resulting in 10⁴ cells/ pellet of mold. The pellets are incubated at 28°C with 95% RH for 7 days and observed for mold growth.

## Claims

1. A method of treating an inanimate surface to provide long-lasting cleaning, including prevention of mold growth on the surface, the method comprising the step of treating the surface with a composition comprising at least 10²cfu/g of bacterial spores wherein the bacterial spores are selected from the group consisting of spores of *Bacillus amyloliquefaciens* strain D747 and a variant thereof, wherein the variant has at least 96% and preferably at least 99% whole genome sequence identity with *Bacillus amyloliquefaciens* strain D747.

2. A method according to claim 1 wherein the variant has 99.8% whole genome sequence identity with *Bacillus amyloliquefaciens* strain D747.

3. A method according to claim 1 wherein the spores comprise spores of *Bacillus amyloliquefaciens* strain QST713.

4. A method according to any of claims 1 or 2 wherein the bacterial spores comprise spores of *Bacillus amyloliquefaciens* strain D747.

5. A method according to any of the preceding claims wherein the bacterial spores comprise the genetic code required for production of the polyketide synthesis enzymes.

6. A method according to any of the preceding claims to provide prevention of mold growth to a surface wherein the surface is a soft or a hard surface and wherein the hard surface is preferably a kitchen or a bathroom surface.

7. A method according to any of the preceding claims wherein the surface is a porous inorganic surface.

8. A method according to any of the preceding claims wherein the bacterial spores are a mixture of bacterial spores comprising at least two, preferably at least three different bacterial strains.

9. Use of bacterial spores selected from the group consisting of spores of *Bacillus amyloliquefaciens* strain D747 and a variant thereof, wherein the variant has at least 96% and preferably at least 99% whole genome sequence identity with *Bacillus amyloliquefaciens* strain D747, to provide long lasting cleaning, including prevention of mold growth on an inanimate surface.

10. Use of bacterial spores according to the preceding claim wherein the spores are selected from the group consisting of spores of *Bacillus amyloliquefaciens* strain D747 and a variant thereof, wherein the variant has at least 98%, preferably at least 99.8% whole genome sequence identity with *Bacillus amyloliquefaciens* strain D747.

11. Use of bacterial spores according to any of claims 9 or 10 to provide prevention of mold growth on an inanimate surface.

12. A cleaning composition, suitable to provide long-lasting cleaning, including prevention of mold growth on an inanimate surface, the composition comprising:
i) bacterial spores selected from the group consisting of spores of *Bacillus amyloliquefaciens* strain D747 and a variant thereof having at least 96% and preferably at least 99% whole genome sequence identity with *Bacillus amyloliquefaciens* strain D747;
ii) a surfactant; and
iii) a perfume.

13. A composition according to the preceding claim comprising at least 10⁴cfu/g of bacterial spores.

14. A composition according to any of claims 12 to 13 wherein the composition is in liquid form.

15. A composition according to any of claims 12 to 14 wherein the composition is an aqueous composition having a pH of from about 4 to about 11 as measured at 20°C.
